# EUROPEAN PATENT SPECIFICATION

(11) **EP 2 065 068 B1**
(45) Date of publication and mention of the grant of the patent: **12.07.2017**
(21) Application number: 07425756.9
(22) Date of filing: 28.11.2007
(51) Int. Cl.: A61M 16/10

(54) **Respiratory filtering device for detecting a dangerous resistance to flow in a flow of gas through a filter**
Beatmungsfiltervorrichtung zum Erkennen eines gefährlichen Strömungswiderstands eines Gasstroms durch einen Filter
Dispositif respiratoire de filtrage pour détecter une résistance dangereuse à l'écoulement dans l'écoulement d'un gaz à travers un filtre

(43) Date of publication of application: 03.06.2009
(73) Proprietor: Covidien AG, 8212 Neuhausen am Rheinfall (CH)
(72) Inventor: Solci, Massimiliano, 42015 Correggio (IT); Pizzo, Samuele, 46035 Revere (IT); Tralli, Stefano, 46022 Felonica (IT)
(74) Representative: Bergadano, Mirko

(56) References cited:
- EP-A- 0 841 083
- WO-A-99/03525
- DE-A1- 2 315 869
- DE-A1- 3 107 974
- DE-C1- 3 832 252
- GB-A- 1 180 937
- GB-A- 2 086 048
- US-A- 6 026 539
- US-A1- 2004 112 273

## Description

The present invention relates to a device for detecting and showing an excessive, and potentially dangerous, resistance to flow in a filter preferably equipped with a heat/moisture exchanger (HME) traversed by a gaseous fluid.

The device forming the subject of the present invention finds advantageous, but non-exclusive, application in the hospital departments of anaesthesia and emergency and intensive care, to which the following description will make explicit reference, without this implying any loss of generality.

As is known, in emergency departments (Intensive Care Unit - ICU), the patient subjected to mechanical ventilation is connected to a ventilation apparatus by way of a respiratory circuit.

A single filter, or a filter provided with an HME element is positioned close to the mouth of the patient in order to protect the patient and/or the ventilation apparatus from any bacterial/viral contamination and, if an HME element is present, in order to heat and humidify the air inhaled by the patient.

The entire system is controlled by the ventilation apparatus, which detects the flows, whether inhaled or exhaled, and the pressures present within the circuit.

In the case of problems, such as pressure losses in the circuit, air leakages, or excessively high pressures, the ventilator apparatus issues an alarm signal to call the attention of an operator in order for him to verify immediately the general conditions of the patient and the state of the system.

Also in the case where there is detected a resistance to flow that is too high for the patient during mechanical ventilation, the ventilation apparatus issues an alarm signal. Normally, the limit of resistance to flow, beyond which an alarm signal is generated, is set manually by the hospital operator. Consequently, owing to an error on the part of the hospital operator, the limit of resistance to the flow beyond which an alarm signal is generated could be set at levels that are excessively high so that the ventilator alarms only when the resistance to flow has already become critical for the patient.

For example, the document No. US 6,035,851 (WALLEN) regards a method and a device for monitoring the conditions of a filter in a ventilator. Two flow detectors and two pressure detectors are provided. A computing unit determines the conditions of the two bacterial filters from the measurement of the flows and pressures at inlet to/outlet from the filters. If the drop in pressure between a point upstream of the filter and a point downstream thereof remains within certain pre-set limits the filter is considered to be still in a good state.

However, the method and device described in US 6, 035, 851 (WALLEN) are complex and costly.

Moreover, in the document WO 99/03525 (PALL CORP at al.) a breathing filter device including a housing that can be sealed without welding is disclosed. Preferably, the device includes a heat and moisture exchange filter element that can be disposed in the housing without compression. However, such breathing filter device is not provided with any device for detecting and showing an excessive resistance to flow in a gaseous fluid through the filter.

Consequently, the aim of the present invention is to provide a device for detecting and showing an excessive, and potentially dangerous, resistance to flow in a filter (preferably equipped with an HME) during traversal by a gaseous fluid, said device being free from the drawbacks described above and, at the same time, being easy and inexpensive to produce.

The present invention provides a respiratory apparatus (100) housing a device (10) for detecting and showing an excessive resistance to flow in a gaseous fluid through a filter preferably equipped with a heat/moisture exchanger; said filter dividing said filtering apparatus into a patient room and a ventilator room. The device is characterized in that it is designed to detect and to show the difference in pressure existing between said ventilator room and said patient room.

In particular, the detection device is housed in a seat made on a wall of a central container of a filtering apparatus at the patient end, the filtering apparatus comprising the filter.

The device is characterized:
- in that it comprises a body closed to the outside world by a window;
- in that the body is divided into two chambers by an elastic membrane; an end wall of the body being provided with a preferably calibrated hole, which connects the first chamber with the patient room; the second chamber being in communication, instead, with the ventilator room by means of a pressure-relief channel; and
- in that it is provided with means for detecting and showing a dangerous resistance to flow in a flow of gas that traverses the filter.

Moreover, the present invention relates to a filtering apparatus, which comprises the aforesaid detection and showing device.

Furthermore, the present invention relates to a ventilation system for intensive care units (ICU), wherein the filtering apparatus, in its turn, comprises the aforesaid detection and showing device.

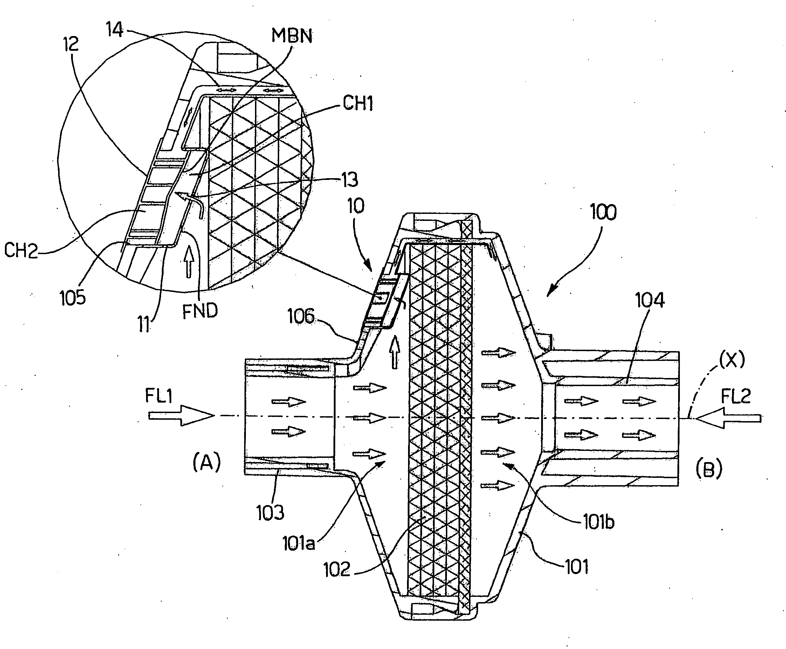

The present invention will now be described with reference to the annexed single figure (accompanied by an enlarged detail thereof), which illustrates a non-limiting example of embodiment of the device.

In the figure, the reference number 10 designates as a whole a device for detecting and showing a dangerous resistance to flow in a gaseous fluid through a filtering apparatus 100 (preferably equipped with an HME) forming the subject of the present invention.

The filtering apparatus 100 comprises a central container 101 occupied in its intermediate part by a filter 102 (preferably equipped with an HME).

The central container is formed by two purposely shaped parts.

Furthermore, the central container 101 is connected to a patient end (A) by way of a first duct 103 and to a ventilator end (B) by means of at least one second duct 104. Filter 102 separates the central container 101 in two rooms, i.e. a patient room 101a and a ventilator room 101b.

As illustrated in the figure, a flow of a gas mixed with water vapour traverses the filter 102, where an exhaled flow FL1 is depurated of possible micro-organisms, and in the case where the filter 102 also comprises an HME, said flow FL1 releases within the HME itself particles of moisture and heat, which will be recovered during a subsequent step of inhalation (see further).

The aforesaid device 10 is housed in a seat 105 made on a wall 106 of the central container 101 at the patient end (A).

In addition, the device 10 comprises a body 11 (see the enlarged detail of the single figure) closed to the outside world by a preferably transparent window 12, which is a continuation of the wall 106 of the central container 101.

The body 11 is divided into two chambers CH1, CH2 by an elastic membrane MBN.

An end wall FND of the body 11 is provided with a preferably calibrated hole 13, which thus connects the first chamber CH1 with the patient end (A). Consequently, in the chamber CH1 there is the gas with a pressure practically equal to the pressure present at the patient end (A).

The second chamber CH2 (separated hermetically from the first chamber CH1 by the elastic membrane MBN), instead, is in communication with the ventilator end (B) by means of a pressure-relief channel 14, made at least partially in the walls of the central container 101, which traverses the filter 102. In other words, within the second chamber CH2 (except for negligible pressure drops due to the traversal of the gas in the pressure-relief channel 14) there is the same pressure as for the gas at the ventilator end (B).

The elastic membrane MBN is characterized by an elasticity such as to react promptly to any variations of pressure that may occur in a filter 102 for adult, paediatric, and neonatal patients.

In normal conditions of operation, an inhaled flow FL2 of gas, that enters the filter 102 coming from the ventilator end (B), traverses said filter 102, reaches the patient. During the subsequent step of exhalation (flow F1), the gas traverses the filter 102 again, yielding moisture and heat to the HME element, obviously in the case where said HME element is present.

If for any reason the filter were to get obstructed, even just partially, the pressure at the patient end (A) starts to increase, and there consequently starts to increase also the pressure within the first chamber CH1, since, as has been said previously, the gas enters this first chamber CH1 through the hole 13. The pressure of the gas in the first chamber CH1 becomes much higher than that of the gas present in the chamber CH2. For this reason, the membrane MBN starts to deflect outwards, preferably adhering to the window 12, which is preferably transparent.

In this very simple way, it is possible to render visible, from outside the central container 101, a danger signal in the form of a symbol, which can be made in such a way as to be displayed gradually as the pressure of the gas in the first chamber CH1 increases with respect to the pressure present in the second chamber CH2, in this way indicating different degrees of danger.

As a result, the hospital operator is able at a glance to assess the state of operation of the filter 102. In effect, the complete appearance of the symbol of danger on the preferably transparent window 12 indicates that the patient is finding some difficulty in breathing, and hence it is necessary to replace the filter 102.

The main advantage of the device described above lies basically in the fact that it is extremely simple to produce and ensures high reliability as regards continuous monitoring of the functionality of the filter.

## Claims

1. A respiratory filtering apparatus (100) housing a device (10) for detecting and showing an excessive resistance to flow in a gaseous fluid through a filter (102); said filter (102) dividing said respiratory filtering apparatus (100) into a patient room (101a) and a ventilator room (101b);
**wherein** said device (10) being designed to detect and to show the difference in pressure existing between said ventilator room (101b) and said patient room (101a);
**and wherein** said device (10) being housed in a seat (105) made on a wall (106) of a central container (101) of a filtering apparatus (100) at the patient end (A), said filtering apparatus (100) comprising said filter (102);
the respiratory filtering apparatus **being characterized by the fact** that said device (10):
- comprises a body (11) closed to the outside world by a window (12); wherein body (11) is divided into two chambers (CH1, CH2) by an elastic membrane (MBN) ; an end wall (FND) of the body (11) being provided with a preferably calibrated hole (13), which connects the first chamber (CH1) with the patient room (101a); the second chamber (CH2) being in communication, instead, with the ventilator room (101b) by means of a pressure-relief channel (14); and
- is provided with means for detecting and showing a dangerous resistance to flow in a flow (FL) of gas that traverses the filter (102); a danger symbol being visible from outside the central container (101) and said danger symbol being associated to said elastic membrane (MBN), which, by deflecting, adheres to said window (12) that can be observed from outside.

2. Respiratory filtering apparatus (100), as claimed in Claim 1, **characterized in that** said danger symbol being made in such a way as to be displayed gradually as the pressure of the gas in the first chamber (CH1) increases with respect to the pressure present in the second chamber (CH2), thus indicating different degrees of danger.

3. Respiratory filtering apparatus (100), as claimed in Claim 2, **characterized in that** said pressure-relief channel (14) is made, at least partially, on at least one of the walls of the central container (101).

4. A ventilation system for intensive care units (ICU), **characterized in that** it comprises at least one respiratory filtering apparatus (100) as claimed in any one of the preceding Claims.

## Patentansprüche

1. Beatmungsfiltervorrichtung (100), in welcher eine Einrichtung (10) zum Erfassen und Anzeigen eines übermäßigen Strömungswiderstands in einem gasförmigen Fluid durch einen Filter (102) angeordnet ist; wobei der Filter (102) die Beatmungsfiltervorrichtung (100) in einen Patienten-Raum (101a) und einen Ventilator-Raum (101b) unterteilt;
wobei die Einrichtung (10) dafür konstruiert ist, den Unterschied im Druck, der zwischen dem Ventilator-Raum (101b) und dem Patienten-Raum (101a) besteht, zu erfassen und anzuzeigen;
und wobei die Einrichtung (10) in einer Aufnahme (105) angeordnet ist, die auf einer Wand (106) eines zentralen Behälters (101) einer Filtervorrichtung (100) am Patienten-Ende (A) gebildet ist, wobei die Filtervorrichtung (100) den Filter (102) umfasst;
wobei die Atmungsfilter-Vorrichtung **dadurch gekennzeichnet ist, dass** die Einrichtung (10):
- einen Körper (11) umfasst, der zur Außenwelt durch ein Fenster (12) geschlossen ist; wobei der Körper (11) durch eine elastische Membran (MBN) in zwei Kammern (CH1, CH2) unterteilt ist; wobei eine Endwand (FND) des Körpers (11) mit einem vorzugsweise kalibrierten Loch (13) versehen ist, welches die erste Kammer (CH1) mit dem Patienten-Raum (101a) verbindet; wobei die zweite Kammer (CH2) stattdessen mittels eines Druckausgleichskanal (14) in Kommunikation mit dem Ventilator-Raum (101b) ist; und
- versehen ist mit Mitteln zum Erfassen und Anzeigen eines gefährlichen Strömungswiderstands in einem Strom (FL) des Gases, das durch den Filter (102) tritt; wobei ein Gefahr-Symbol von außerhalb des zentralen Behälters sichtbar ist und wobei das Gefahr-Symbol der elastischen Membran (MBN) zugeordnet ist, welche durch Auslenkung an dem Fenster (12) anhaftet, welches von außen beobachtbar ist.

2. Beatmungsfiltervorrichtung (100) nach Anspruch 1, **dadurch gekennzeichnet, dass** das Gefahr-Symbol derart ausgebildet ist, allmählich angezeigt zu werden, wenn der Druck des Gases in der ersten Kammer (CH1) mit Bezug auf den in der zweiten Kammer (CH2) herrschenden Druck zunimmt, so dass unterschiedliche Abstufungen der Gefahr angezeigt werden.

3. Beatmungsfiltervorrichtung (100) nach Anspruch 2, **dadurch gekennzeichnet, dass** der Druckausgleichskanal (14) wenigstens teilweise auf wenigstens einer der Wände des zentralen Behälters (101) ausgebildet ist.

4. Belüftungssystem für Intensivpflege-Einheiten (I-CU), **dadurch gekennzeichnet, dass** es wenigstens eine Beatmungsfiltervorrichtung (100) nach einem der vorangehenden Ansprüche umfasst.

## Revendications

1. Appareil de filtrage respiratoire (100) contenant un dispositif (10) pour détecter et montrer une résistance excessive à l'écoulement dans un fluide gazeux à travers un filtre (102); ledit filtre (102) divisant ledit appareil de filtrage respiratoire (100) en une région de patient (101a) et une région de ventilateur (101b);
dans lequel ledit dispositif (10) est conçu de manière à détecter et à montrer la différence de pression qui existe entre ladite région de ventilateur (101b) et ladite région de patient (101a); et
dans lequel ledit dispositif (10) est logé dans un siège (105) formé sur une paroi (106) d'un récipient central (101) d'un appareil de filtrage (100) à l'extrémité de patient (A), ledit appareil de filtrage (100) comprenant ledit filtre (102);
l'appareil de filtrage respiratoire étant **caractérisé par le fait que** ledit dispositif (10):
- comprend un corps (11) isolé du monde extérieur par une fenêtre (12); dans lequel le corps (11) est divisé en deux chambres (CH1, CH2) par une membrane élastique (MBN); une paroi d'extrémité (FND) du corps (11) comportant un trou de préférence calibré (13), qui relie la première chambre (CH1) à la région de patient (101a); la seconde chambre (CH2) étant au contraire en communication avec la région de ventilateur (101b) par l'intermédiaire d'un canal de détente de pression (14); et
- est pourvu de moyens pour détecter et montrer une résistance dangereuse à l'écoulement dans un écoulement (FL) de gaz qui traverse le filtre (102); un symbole de danger étant visible depuis l'extérieur du récipient central (101), et ledit symbole de danger étant associé à ladite membrane élastique (MBN) qui, par déflexion, adhère à ladite fenêtre (12) qui peut être observée depuis l'extérieur.

2. Appareil de filtrage respiratoire (100) selon la revendication 1, **caractérisé en ce que** ledit symbole de danger est formé de manière à être affiché graduellement lorsque la pression du gaz dans la première chambre (CH1) augmente par rapport à la pression présente dans la seconde chambre (CH2), indiquant ainsi différents degrés de danger.

3. Appareil de filtrage respiratoire (100) selon la revendication 2, **caractérisé en ce que** ledit canal de détente de pression (14) est formé, au moins en partie, sur au moins une des parois du récipient central (101).

4. Système de ventilation pour des unités de soins intensifs (ICU), **caractérisé en ce qu'**il comprend au moins un appareil de filtrage respiratoire (100) selon l'une quelconque des revendications précédentes.
